(19) **Europäisches Patentamt**
**European Patent Office**
**Office européen des brevets**

(11) **EP 3 154 617 B1**

(12) # EUROPEAN PATENT SPECIFICATION

(45) Date of publication and mention
of the grant of the patent:
**15.07.2020 Bulletin 2020/29**

(51) Int Cl.:
**A61M 16/20** (2006.01)       **A61M 16/00** (2006.01)

(21) Application number: **14739555.2**

(86) International application number:
**PCT/IB2014/062234**

(22) Date of filing: **14.06.2014**

(87) International publication number:
**WO 2015/189664 (17.12.2015 Gazette 2015/50)**

(54) **VOLUME DIVIDER AND METHOD OF RESPIRATORY GAS DIVISION**

VOLUMENTRENNER UND VERFAHREN ZUR ATEMGASTRENNUNG

DIVISEUR DE VOLUME ET PROCÉDÉ DE DIVISION DE GAZ RESPIRATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.04.2017 Bulletin 2017/16**

(73) Proprietor: **Instytut Biocybernetyki I Inzynierii Biomedycznej**
**Im. Macieja Nalecza Pan**
**02-109 Warszawa (PL)**

(72) Inventors:
• **DAROWSKI, Marek**
**PL-02-070 Warszawa (PL)**
• **KOZARSKI, Maciej**
**PL-05-806 Granica (PL)**
• **STANKIEWICZ, Barbara**
**PL-05-806 Komorów (PL)**
• **MICHNIKOWSKI, Marcin**
**PL-02-443 Warszawa (PL)**
• **PALKO, Krzysztof Jakub**
**PL-02-156 Warszawa (PL)**
• **ZIELINSKI, Krzysztof**
**PL-01-242 Warszawa (PL)**

(74) Representative: **Patpol Kancelaria Patentowa Sp. z o.o.**
**Nowoursynowska 162J**
**02-776 Warszawa (PL)**

(56) References cited:
**EP-A2- 0 118 850       WO-A1-2006/067822**
**US-A- 4 674 496       US-A- 5 752 507**

• **Darowski M. et al: "A New Control Solution for Independent Synchronous Ventilation of Lungs", Biocybernetics and Biomedical Engineering, vol. 30, no. 2 2010, pages 29-39, XP002728316, Retrieved from the Internet: URL:http://www.ibib.waw.pl/bbe/bbefulltext /bbe_30_2_029_ft.pdf [retrieved on 2014-08-07]**

## Description

The invention concerns a gas mixture volume divider. More particularly, the object of the invention is an automatic inspiratory gas mixture volume divider supplied from a medical ventilator into the lungs.

Inspiratory mixture volume dividers are used during artificial ventilation of lungs in two cases: separate ventilation of lungs of one patient or ventilation of two patients by means of just one medical ventilator. The first case takes place when pathology (e.g. cancerous lesions) in one of lungs causes that better results of respiratory therapy can be obtained by ventilating each of the patient's lungs independently, supplying therein varied and controlled volumes of the inspiratory gas. Also during cardiothoracic surgery the possibility of controlled variation of ventilation of each of the lungs makes the surgery easier. A classic, but very complicated and expensive solution is to use then two synchronized medical ventilators. However ventilation of two patients by means of one medical ventilator makes sense and is relevant when, for unpredicted events threatening human health and life (e.g. epidemic outbreak, flood or gaseous terrorist attack), the number of medical ventilators available at the location of the events mentioned above is smaller than the number of people requiring artificial ventilation of lungs. There are known various technical solutions for division of volume of inspiratory gas supplied from a medical ventilator. In general, these are based on application of simple pneumatic resistors arranged in two inspiratory branches guiding the inspiratory mixture from the medical ventilator.

From Polish patent description no. 179784 there is known also a system for selective ventilation of lungs, wherein, in one solution a flow intensity regulator in one or two inspiratory branches, and in another solution a regulator of flow intensity ratio of these flows has been applied. The advantage of these solutions is an increased accuracy of inspiratory volumes division than in the case of using for this purpose simple pneumatic resistors. In the case of different flow velocity profiles in two inspiratory branches, occurring when resistances of the inspiratory branches and/or susceptibilities of lungs are different, adjustment of intensities of inspiratory flows or their ratio not only does not provide accurate and stable ventilation control for each of the lungs, but it can even be aleatory. Another significant drawback and limitation of the system for selective ventilation of lungs, presented in the patent description no. 179784, is that it is adapted for constant flow ventilators, which are currently superseded by constant pressure ventilators. Further, it does not provide sufficient control of expiratory pressure.

Publication Darowski M. et al: "A New Control Solution for Independent Synchronous Ventilation of Lungs", Biocybernetics and Biomedical Engineering, vol. 30, no. 2, 2010, discloses an inspiratory volume divider comprising at least two inspiratory lines ending with inspiratory branches and comprising one-way valves and at least two expiratory lines ending with expiratory branches and comprising one-way valves, wherein the inspiratory branches and the expiratory branches are in pairs combined with each other in at least two inspiratory-expiratory pairs, while the initial portions of the inspiratory lines are connected to a valve divider, wherein the valve divider is provided with a control input, and into the inspiratory lines after the valve divider there are interconnected gas volume measurement systems, the output signals thereof are delivered to a controller, the output signals thereof are delivered to a controller, the output signal thereof is connected to the volume divider's control input.

The aim of the invention is to solve the drawbacks of solutions known in the state of the art mentioned above and related problems. This is not a trivial task, because in the publication of R.D. Branson, T.C. Blakeman, B. RH. Robinson, J.A. Johanngman entitled "Use of a Single Ventilator to Support 4 Patients: Laboratory Evaluation of a Limited Concept, it was pointed that currently known division techniques for inspiratory gas from a medical ventilator do not qualify for application with patients due to high unreliability.

An inspiratory gas volume divider according to the invention comprises at least two inspiratory lines ending with inspiratory branches and comprising one-way valves, and at least two expiratory lines ending with expiratory branches and comprising one-way valves, wherein the inspiratory branches and the expiratory branches are combined in pairs with each other in at least two inspiratory-expiratory pairs, while the initial portions of the inspiratory lines are connected to the valve divider. The valve divider is provided with a control input, wherein the fragments of inspiratory lines in the valve divider are in the form of elastic tubes deformable by means of a moveable element for controlling gas volume in the inspiratory lines downstream of the valve divider; inspiratory gas volume measurement systems are interconnected into the inspiratory lines downstream of the valve divider, wherein the output signals of the inspiratory gas volume measurement systems are delivered to the controller for controlling the inlet control means of the valve divider and the output signals of the controller (22) are delivered to the volume divider's control input.

The application of volume meters allows for an accurate gas volume division, independent from resistances of respiratory tracts. Further, the system with volume meters is less susceptible to self-excitation in the case of partially blocked respiratory tracts. Therefore the proposed system is suitable for application with constant pressure ventilators while providing control over the actual volume of gas supplied into the lungs. The inspiratory volume divider according to the invention has substantially superior performance, in comparison to the solution according to the patent no. 179784. Including systems measuring current volume of air supplied to the lungs separately in each inspiratory line and including these converters in the automatic system controlling the division of volumes supplied to both lungs by means of controlled valve dividing the main gas stream makes the volume division independent from variations of mechanical parameters of both lungs. This case assures that a total volume of gas set by a physician will be supplied to the lungs with defined division between both lungs. Applying, like in solution according to patent no. 179784, the principle of division of gas by fixing constant instantaneous proportion of streams as opposed to volumes, leads simultaneously to the desired division of volume of gas supplied to the left and the right lung only in particular cases.

Preferably the inspiratory gas volume divider is provided with a monitoring circuit and there are expiratory gas volume meters included in the expiratory lines, wherein the output signals of the expiratory gas volume meters are delivered to the monitoring circuit.

Preferably the valve divider is controlled by an electric motor.

More preferably, the output ends are pneumatic.

Even more preferably, the movable element is a pin which is mounted on a slider having rolls of a straight line mechanism with bearings in the housing.

Also preferably, there is a leadscrew associated with the threaded hole of the straight line mechanism's rolls, driven by the motor.

The motor is preferably a direct current motor.

Also preferably the motor is attached to an amplifier-power driver.

More preferably the gas volume measurement systems are integrated with the valve divider.

Preferably the systems measuring volume are digital meters providing digital measurement signal and the controller is provided with digital signal processing means adapted to convert the measurement results into the control signal for the valve divider. Such solution makes it possible to incorporate advanced processing techniques and settings with improved precision.

Preferably the expiratory branches are provided with positive end-expiratory pressure (PEEP) valves. Such valve opens only under condition that a minimal value of pressure exists at its input. Its use prevents the risk of complete pulmonary alveoli collapse in the expiration phase. Furthermore, preferably the divider is provided with a monitoring circuit, and in the expiratory branches there are included volume meters and pressure meters, the output signals thereof being connected to the monitoring circuit.

Selective ventilation of lungs is applied, inter alia, when mechanical properties, i.e. resistance of respiratory tracts, susceptibility of pulmonary alveoli, of both lungs are significantly different. As a result, inspiratory gas flow in each of the two inspiratory lines obtains different instantaneous value and, as a consequence, different flow velocity profiles are dealt with. Only the solution according to the invention provides proper information about the gas volume being delivered to the lungs at every time instant of the inspiration phase. Such information is not provided by solutions known in the state of the art, wherein flow intensities or their ratio are subjected to measurement.

The method of inspiratory gas volume division according to the invention is used in a divider as mentioned above and as defined in the appended claim 1, the divider comprising at least two expiratory lines and two inspiratory lines connected to a controlled divider provided with a control input. The method comprises the step of setting the requested inspiratory gas volume division in the valve divider, next the step of measuring and the step of subsequent automatic setting of the division. In this way the feedback loop, providing division in relevance to the measurement, is closed. In the step, gas volume in each of the inspiratory lines is measured, and the measurement signal is converted in an automatic controller into the valve divider's control signal.

Preferably the method according to the invention further comprises the step of measuring gas volume in expiratory lines and also preferably a step of measuring pressure and displaying the values at a monitoring circuit.

The object of the invention has been shown in embodiments in the drawings, wherein Fig. 1 is a schematic diagram of the divider according to the invention, Fig. 2 shows an embodiment of the invention having a valve divider provided with acceleration sensor, Fig. 3 shows an alternative valve divider, and Fig. 4 is a block diagram of the divider according to the invention having such valve divider.

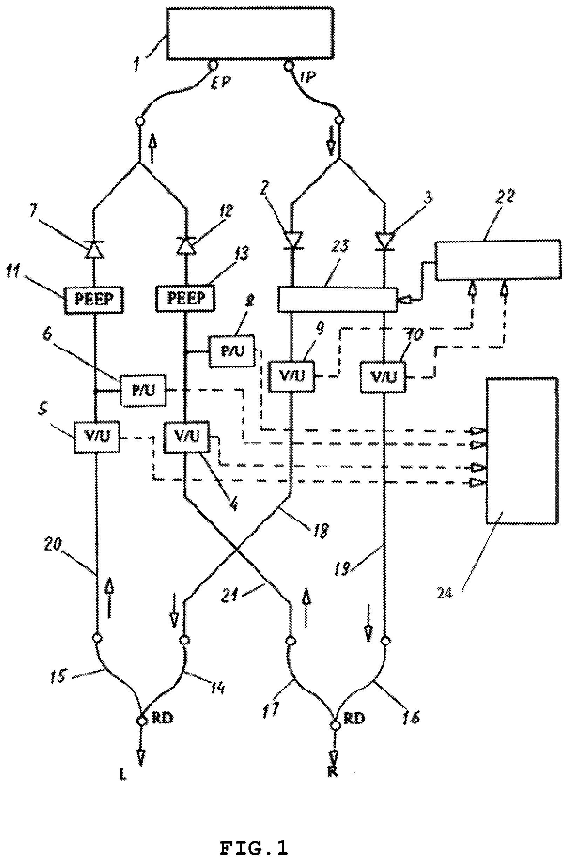

The object of the invention has been described in embodiments in the drawings, wherein Fig. 1 is a schematic diagram of the apparatus for independent ventilation of two lungs with selective application of positive end-expiratory pressures.

The volume divider comprises two parallel inspiratory lines 18 and 19, and two parallel expiratory lines 20 and 21. The inspiratory and expiratory lines end with branches, respectively, inspiratory 14 and 16, and expiratory 15 and 17, combined in inspiratory-expiratory pairs. In each pair the inspiratory branch 14 with the expiratory branch 15 and the inspiratory branch 16 with the expiratory branch 17 are connected to each other. The common point of the branches inspiratory 14 and expiratory 15 is connected to one endotracheal tube conduit RD and the common point of the branches inspiratory 16 and expiratory 17 is connected to another endotracheal tube conduit RD.

In the combined in parallel inspiratory lines 18 and 19 there are respectively arranged one-way pneumatic valves 2 and 3. In the expiratory lines 20 and 21 there are pressure indicators 6 and 8. In the combined in parallel expiratory lines 20 and 21 there are arranged one-way pneumatic valves 7 and 12.

The one-way valve 7 is connected in series with the volume meter 5, positive end-expiratory pressure valve 11 and the expiratory branch 15 and the one-way valve 12 is connected in series with the volume meter 4 and the expiratory branch 17.

The common input of one-way valves 2 and 3 is connected to the inspiratory channel IP of the medical ventilator 1, the common output of the one-way valves 7 and 12 is connected to the expiratory channel EP of the medical ventilator 1. Outputs of valves 2 and 3 are connected to the volume valve divider 23 controlled via the controller 22 to which the measurement signals from the volume measuring systems 9, 10 are delivered. In this way, the loop regulating both division as well as magnitude of volume of the inspiratory gas supplied to each of the lungs is closed.

As the system measuring the gas volume 9, 10 there can be used a flow system with analogue or digital integrating circuit, providing a signal related to the volume of gas introduced into the patient's lungs. As the controller 22 there can be then used analogue circuits, but also a microcontroller or an FPGA device. In digital solutions it is possible to implement more advanced techniques of signal processing and obtain settings with improved accuracy.

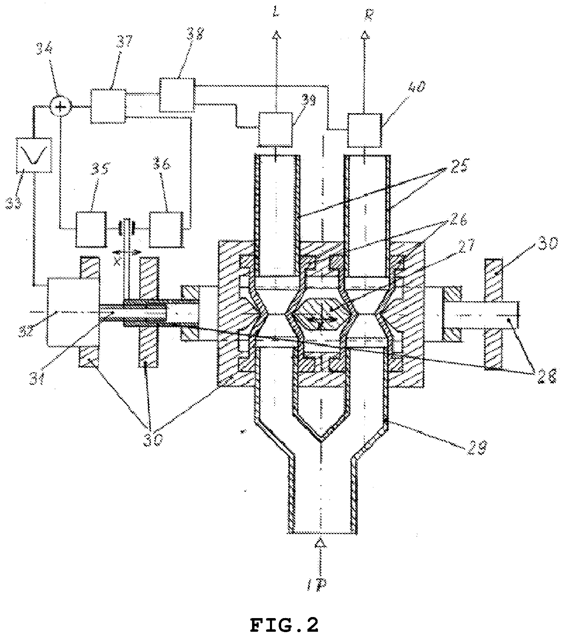

In an alternative embodiment, the systems measuring gas volume 9, 10 are integrated with electrically controlled valve divider shown in Fig 2. In the stationary housing 30 of the valve there are mounted pneumatic output ends 25 and elastic, preferably made of rubber, tubes 26 deformed by means of a moveable pin 27 mounted on a slider having rolls 28 of a straight line mechanism with bearings in the housing 30. With the threaded hole of the straight line mechanism's rolls 28 there is associated a leadscrew 31 driven by an electric motor 32, preferably a direct current motor, attached to an amplifier - power driver 33. The amplifier control signal comes from a node 34, wherein two signals are being summed: a signal $\frac{d^2x}{dt^2}$ from the acceleration sensor 35 and a signal from the first controller's output 37, preferably a PID controller, which together with a linear converter 36 of displacement forms a circuit controlling the slider's 28 position x. To the first input of the controller 37 there is delivered an input signal from the converter 36, while to its second input there is delivered the output signal from the second controller 38. Controller's 38 inputs are connected to electric outputs of the converters 39 and 40 of instantaneous value of gas volume supplied to the left L and the right R lung. The described control circuit is cascaded, wherein the inner control loop for displacement x allow, inter alia, to pre-set the pin's 27 position in a position assuring a preliminary division of streams directed to the left L and the right R lung. The second end of elastic tubes 26 is connected to a tee junction 29, to which the gas stream from the medical ventilator is delivered. It should be emphasised that in the described embodiment of the valve divider the disposable element is the whole pneumatic tract, thus the tee junction 29, rubber tubes 26 and output ends 25.

The operation of the device according to the invention is as follows. In the inspiration phase, the gas flowing out of the inspiratory canal IP of the ventilator 1 is being divided into two inspiratory lines 18 and 19, and flows through the one-way pneumatic valves 2 and 3, and through inspiratory gas volume meters 9 and 10. The gas flowing through the inspiratory line 18 is directed via the inspiratory branch 14 and one of endotracheal tube conduits to one of the lungs, because the expiratory port EP of the ventilator is closed during the inspiration phase. The gas flowing through the inspiratory line 19 is directed via the inspiratory branch 16 and the second endotracheal tube conduit RD to the second lung.

The increase of pressure in the inspiratory branches 14 and 16 in the inspiration phase and the expiration phase is measured and respectively indicated by pressure indicators 6 and 8. In the expiration phase the inspiration canal IP of the ventilator is closed and the expiration canal EP of the ventilator is open, causing flowing out of gases from lungs. And so the gas flows from one of the lungs through one of the endotracheal tube conduits RD, through the expiratory branch 15, the volume meter 5, the positive end-expiratory pressure valve PEEP 11, the one-way pneumatic valve 7, to the expiratory canal EP of the ventilator. From the other lung the gas flows through the second endotracheal tube conduit RD through the expiratory branch 17, the volume meter 4, the one-way pneumatic valve 12, to the expiratory canal EP of the ventilator. The one-way valves 2, 3, 7, 12 arranged in strictly defined locations in the considered system of connections of the device determine the flow of gases between the medical ventilator and the lungs in respective phases of the respiratory cycle - during inspiration and expiration - such as described above, this way making possible both independent ventilation of each lung and obtaining positive expiratory pressure only in one of the lungs by means of the PEEP valve 11 or the PEEP valve 13. By means of the volume ratio controller 22 and the valve divider 23 the flow of gas in one of the inspiratory lines is decreased, and increased in the other inspiratory line simultaneously.

The apparatus according to the invention further comprises the positive end-expiratory pressure valve PEEP 13, by means of which the positive end-expiratory pressure is set in the expiratory branch 17, thus also in the lung connected thereto via the endotracheal tube conduit.

The above solution also makes possible, except of the independent ventilation of each lung, obtaining of positive end-expiratory pressures independently in both lungs.

The application of the monitoring circuit 24 and displaying thereon the indications from the meters of pressure and volume in the expiratory lines provides the possibility of enhanced control of the patient's condition and the course of ventilation and also displaying relevant information for the physician. In particular, the subjected to measurement values can be digitized, and the monitoring circuit can be constituted by a typical unit provided with a central unit, memory, data receivers and a display.

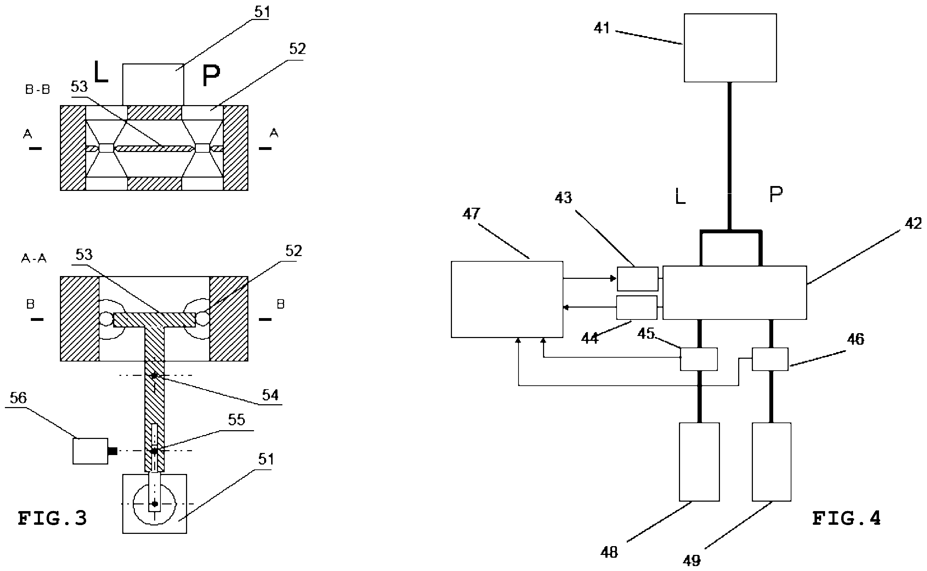

An alternative construction of the valve divider is shown in Fig. 3. On the stepper motor's 51 shaft there is arranged a bar with a pin 55, which enters the opening of a moveable plate 53, rotating on the axis 54. The upper portion of the movable plate 53 clamps the elastic passages 52, arranged in the line of flow for the left L and the right P lung. When the axis of the stepper motor 51 rotates right, the pin 55 slides right, sliding the bottom portion of the moveable plate 53 also right. As a result, the plate 53 rotates around the axis 54 and its upper portion slides left, narrowing the left passage L and opening the right passage P. The degree of opening of the passages depends on the rotation angle of the motor's axis. When the motor's axis 51 rotates left, the right passage P is being narrowed and the left one L is being opened. The position sensor 56 is used to pre-set the control system, that is, to detect the position of zero.

A block diagram of the arrangement of the inspiratory gas divider with such valve divider is shown in Fig. 4 together with indicated medical ventilator 41 to which it is connected to. The flow generated by the medical ventilator 41 is delivered to the left and to the right passage of the controller 42. The flow after the passages, the left and the right, is measured by means of volume sensors 45 and 46. The control system 47 sets the position of the driving motor 43 according to the applied volume division algorithm.

It will be apparent to a person skilled in the art that the present invention can be implemented in multiple different ways with the use of numerous components, sensors, meters and signal processing algorithms known in the state of the art, obtaining a solution falling within the scope of protection as defined by the attached claims.

**Claims**

1. An inspiratory gas volume divider comprising at least two inspiratory lines (18, 19) ending with inspiratory branches (14,16) and comprising one-way valves (2,3), and at least two expiratory lines (20, 21) ending with expiratory branches (15,17) and comprising one-way valves (7,12), wherein the inspiratory branches (14,16) and the expiratory branches (15,17) are combined in pairs with each other in at least two inspiratory-expiratory pairs, while the initial portions of the inspiratory lines are connected to a valve divider (23), **characterised in that** the valve divider (23) is provided with a control input, wherein the fragments of inspiratory lines (18, 19) in the valve divider (23) are in the form of elastic tubes (26) deformable by means of a moveable element (27) for controlling gas volume in the inspiratory lines (18, 19) downstream of the valve divider (23); inspiratory gas volume measurement systems (9,10) are interconnected into the inspiratory lines (18,19) downstream of the valve divider (23), wherein the output signals of the inspiratory gas volume measurement systems (9,10) are delivered to a controller (22) for controlling the inlet control means of the valve divider (23) and the output signals of the controller (22) are delivered to the volume divider's control input.

2. The inspiratory gas volume divider according to claim 1, **characterized in that** it is provided with a monitoring circuit (24) and **in that** there are expiratory gas volume meters (4,5) included in the expiratory lines (20,21), wherein the output signals of the expiratory gas volume meters (4,5) are delivered to the monitoring circuit (24).

3. The inspiratory gas volume divider according to claim 1, **characterized in that** the valve divider (23) is controlled by an electric motor (32).

4. The inspiratory gas volume divider according to any preceding claim, **characterized in that** the valve divider (23) comprises output ends (25) which are pneumatic.

5. The inspiratory gas volume divider according to any preceding claim, **characterized in that** the movable element (27) is a pin which is mounted on a slider having rolls (28) of a straight line mechanism with bearings in the housing (30).

6. The inspiratory gas volume divider according to claim 3 and 5, **characterized in that** there is a leadscrew (31) associated with the threaded hole of the straight line mechanism's rolls (28), driven by the motor (32).

7. The inspiratory gas volume divider according to claim 6, **characterized in that** the motor (32) is a direct current motor.

8. The inspiratory gas volume divider according to claim 3 or 6, **characterized in that** the motor (32) is attached to an amplifier-power driver (33).

9. The inspiratory gas volume divider according to any preceding claim, **characterized in that** the gas volume measurement systems (9,10) are integrated with the valve divider (23).

10. The divider according to any preceding claim, **characterised in that** the volume measurement systems are digital meters providing a digital measurement signal, and the controller (22) is provided with digital signal processing means adapted to convert the measurement results into the valve divider's control signal.

11. The inspiratory gas volume divider according to any preceding claim, **characterised in that** the expiratory branches are provided with positive end-expiratory pressure valves PEEP (11,13).

12. The inspiratory gas volume divider according to claim 11 when dependent on claim 2, **characterised in that** in the expiratory branches there are included pressure meters (6,8) the output signals thereof being connected to the monitoring circuit (24).

13. A method of inspiratory gas volume division in a divider according to any of the preceding claims, comprising the step of setting the requested inspiratory gas volume division, the step of measuring, and the step of subsequent resetting in feedback loop, **characterised in that** subjected to the measurement is the volume of gas in each of the inspiratory lines, and the measurement signal in automatic controller is converted into the valve divider's control signal.

14. The method of inspiratory gas volume division according to claim 13, **characterised in that** it further comprises the step of measuring the volume of gas in the expiratory lines and displaying the indication on the monitoring circuit.

15. The method of inspiratory gas volume division according to claim 13 or 14, **characterised in that** it further comprises the step of measuring the pressure of gas in the expiratory lines and displaying the indication on the monitoring circuit (24).

## Patentansprüche

1. Ein Verteiler des inspiratorischen Gasstroms, umfassend mindestens zwei inspiratorische Leitungen (18, 19), die mit inspiratorischen Verteilungen (14, 16) enden sowie Einwegventile (2, 3) umfassen, und mindestens zwei exspiratorische Leitungen (20, 21), die mit exspiratorischen Verteilungen (15, 17) enden und Einwegventile (7, 12) umfassen, wobei die inspiratorischen Verteilungen (14, 16) und die exspiratorischen Verteilungen (15, 17) paarweise miteinander als mindestens zwei inspiratorisch-exspiratorische Paare gekoppelt sind, während die Anfangsabschnitte der inspiratorischen Leitungen an einen Ventilteiler (23) angeschlossen sind, **dadurch gekennzeichnet, dass** der Ventilverteiler (23) mit einem Steuereingang versehen ist, wobei die Abschnitte von inspiratorischen Leitungen (18, 19) im Ventilverteiler (23) in Form von elastischen Schläuchen (26) ausgeführt sind, die mittels eines beweglichen Teils (27) zur Steuerung des fließenden Gasstroms in den inspiratorischen Leitungen (18, 19) nach dem Ventilverteiler (23) verformbar sind;
Messsysteme (9, 10) des inspiratorischen Gasstroms in den inspiratorischen Leitungen (18, 19) nach dem Ventilverteiler (23) integriert sind, wobei die Ausgangssignale von Messsystemen (9, 10) des inspiratorischen Gasstroms zu einer Steuereinheit (22) zur Steuerung der Einlässe des Ventilverteilers (23) und die Ausgangssignale der Steuereinheit (22) zum Steuereingang des Volumenverteilers geleitet werden.

2. Der Verteiler des inspiratorischen Gasstroms nach Anspruch 1, **dadurch gekennzeichnet, dass** er mit einem Überwachungsschaltkreis (24) versehen ist und dass in den exspiratorischen Leitungen (20,21) Messer (4, 5) des exspiratorischen Gasstroms eingebaut sind, wobei die Ausgangssignale der Messer (4, 5) des exspiratorischen Gasstroms an den Überwachungsschaltkreis (24) geleitet werden.

3. Der Verteiler des inspiratorischen Gasstroms nach Anspruch 1, **dadurch gekennzeichnet, dass** der Ventilverteiler (23) durch einen Elektromotor (32) gesteuert wird.

4. Der Verteiler des inspiratorischen Gasstroms nach einem der vorgenannten Ansprüche, **dadurch gekennzeichnet, dass** der Ventilverteiler (23) Abtriebe (25) aufweist, die pneumatisch sind.

5. Der Verteiler des inspiratorischen Gasstroms nach einem der vorgenannten Ansprüche, **dadurch gekennzeichnet, dass** der bewegliche Teil (27) als ein Stift ausgebildet ist, der auf einem Gleitstück mit Rollen (28) eines geradlinigen Mechanismus mit Lagern im Gehäuse (30) montiert ist.

6. Der Verteiler des inspiratorischen Gasstroms nach Anspruch 3 und 5, **dadurch gekennzeichnet, dass** eine Leitspindel (31) vorhanden ist, die mit einer Gewindebohrung der Rollen (28) des geradlinigen Mechanismus gekoppelt und vom Motor (32) angetrieben wird.

7. Der Verteiler des inspiratorischen Gasstroms nach Anspruch 6, **dadurch gekennzeichnet, dass** der Motor (32) ein Gleichstrommotor ist.

8. Der Verteiler des inspiratorischen Gasstroms nach Anspruch 3 oder 6, **dadurch gekennzeichnet, dass** der Motor (32) an einen Treiber mit Verstärker (33) angeschlossen ist.

9. Der Verteiler des inspiratorischen Gasstroms nach einem der vorgenannten Ansprüche, **dadurch gekennzeichnet, dass** die Gasstrom-Messsysteme (9,10) mit dem Ventilverteiler (23) integriert sind.

10. Der Verteiler nach einem der vorgenannten Ansprüche, **dadurch gekennzeichnet, dass** die Gasstrommesssysteme digitale Messgeräte sind, die di-

**11**             **EP 3 154 617 B1**        **12**

gitale Messsignale senden, und dass die Steuereinheit (22) mit Mitteln der digitalen Signalverarbeitung versehen ist, die die ankommenden Messergebnisse in ein Steuersignal des Ventilverteilers umwandeln können.

11. Der Verteiler des inspiratorischen Gasstroms nach einem der vorgenannten Ansprüche, **dadurch gekennzeichnet, dass** die exspiratorischen Verteilungen mit PEEP-Ventilen (11,13) zur Messung des positiven endexspiratorischen Drucks ausgestattet sind.

12. Der Verteiler des inspiratorischen Gasstroms nach Anspruch 11 sofern abhängig von Anspruch 2, **dadurch gekennzeichnet,**
**dass** in den exspiratorischen Verteilungen Druckmesser (6,8) enthalten sind, deren Ausgangssignale an den Überwachungsschaltkreis (24) angeschlossen sind.

13. Ein Verfahren zur Verteilung des inspiratorischen Gasstroms im Verteiler nach einem der vorgenannten Ansprüche, das folgende Schritte umfasst: Einstellen der Sollverteilung des inspiratorischen Gasstroms, Messen, anschließendes Zurücksetzen in einer Rückkopplungsschleife, **dadurch gekennzeichnet, dass** der Gasstrom in jeder der inspiratorischen Leitungen gemessen wird, und das Messsignal in einer automatischen Steuereinheit in das Steuersignal für den Ventilverteiler umgewandelt wird.

14. Ein Verfahren zur Verteilung des inspiratorischen Gasstroms nach Anspruch 13, **dadurch gekennzeichnet, dass** es darüber hinaus den Schritt des Messens des Gasstroms in den exspiratorischen Leitungen und des Anzeigens am Überwachungsschalkreis umfasst.

15. Ein Verfahren zur Verteilung des inspiratorischen Gasstroms nach Anspruch 13 oder 14, **dadurch gekennzeichnet, dass** es darüber hinaus den Schritt des Messens des Gasdrucks in den exspiratorischen Leitungen und des Anzeigens am Überwachungsschalkreis (24) umfasst.

**Revendications**

1. Un diviseur de volume de gaz inspiratoire comprenant au moins deux lignes d'inspiration (18, 19) se terminant par des branches inspiratoires (14, 16) et comprenant des valves unidirectionnelles (2, 3), et au moins deux lignes d'expiration (20, 21) se terminant par des branches expiratoires (15, 17) et comprenant des valves unidirectionnelles (7, 12), dans lequel les branches inspiratoires (14, 16) et les branches expiratoires (15, 17) forment des paires combinées les unes aux autres en au moins deux paires inspiratoires-expiratoires, tandis que les parties initiales des lignes d'inspiration sont connectées à la valve de diviseur (23), **caractérisé en ce que**
la valve de diviseur (23)est munie d'une entrée de contrôle, où les fragments de lignes d'inspiration (18, 19) dans la valve de diviseur (23)sont sous la forme de tubes élastiques (26) déformables au moyen d'un élément mobile (27) pour contrôler le volume de gaz dans les lignes d'inspiration (18, 19) en aval de la valve de diviseur (23);
des systèmes de mesure de volume de gaz inspiratoire (9, 10) sont interconnectés dans les lignes d'inspiration (18, 19) en aval de la valve de diviseur (23); où les signaux de sortie des systèmes de mesure du volume de gaz inspiratoire (9, 10) sont envoyés au dispositif de commande (22) pour commander les moyens de commande d'entrée de la valve de diviseur (23) et les signaux de sortie du dispositif de commande (22) sont envoyés à l'entrée de contrôle du diviseur de volume.

2. Le diviseur de volume de gaz inspiratoire selon la revendication 1, **caractérisé en ce qu'**il est muni d'un circuit de surveillance (24) et **en ce que** des débitmètres de gaz expiratoire (4,5) sont inclus dans les lignes d'expiration (20,21), où des signaux de sortie des débitmètres de gaz expiratoire (4,5) sont envoyés au circuit de surveillance (24).

3. Le diviseur de volume de gaz inspiratoire selon la revendication 1, **caractérisé en ce que** la valve de diviseur (23) est contrôlé par un moteur électrique (32).

4. Le diviseur de volume de gaz inspiratoire selon l'une quelconque des revendications précédentes, **caractérisé en ce que** la valve de diviseur (23) comprend des extrémités (25) de sortie qui sont pneumatiques.

5. Le diviseur de volume de gaz inspiratoire selon l'une quelconque des revendications précédentes, **caractérisé en ce que** l'élément mobile (27) est un pivot qui est monté sur un coulisseau ayant des rouleaux (28) d'un mécanisme de ligne droite avec des roulements dans le boîtier (30).

6. Le diviseur de volume de gaz inspiratoire selon les revendications 3 et 5, **caractérisé en ce qu'**il y a une vis-mère (31) associée au trou fileté des rouleaux (28) du mécanisme de ligne droite, entraînée par le moteur (32).

7. Le diviseur de volume de gaz inspiratoire selon la revendication 6, **caractérisé en ce que** le moteur (32) est un moteur à courant continu.

**7**

**8.** Le diviseur de volume de gaz inspiratoire selon la revendication 3 ou 6, **caractérisé en ce que** le moteur (32) est fixé à un membre d'entraînement (33) d'amplificateur.

**9.** Le diviseur de volume de gaz inspiratoire selon l'une quelconque des revendications précédentes, **caractérisé en ce que** les systèmes de mesure de volume de gaz (9, 10) sont intégrés à la valve de diviseur (23).

**10.** Le diviseur selon l'une quelconque des revendications précédentes, **caractérisé en ce que** les systèmes de mesure de volume sont des compteurs numériques fournissant un signal de mesure numérique, et le dispositif de commande (22) est muni de moyens de traitement de signal numérique adaptés pour convertir les résultats de mesure en signal de commande de valve de diviseur.

**11.** Le diviseur de volume de gaz inspiratoire selon l'une quelconque des revendications précédentes, **caractérisé en ce que** les lignes d'expiration sont munies de valves de pression positive en fin d'expiration PEEP (11, 13).

**12.** Le diviseur de volume de gaz inspiratoire selon la revendication 11 lorsqu'elle dépend de la revendication 2, **caractérisé en ce que**, dans les lignes d'expiration, il y a des manomètres (6,8) dont les signaux de sortie sont connectés au circuit de surveillance (24).

**13.** Un procédé de division du volume de gaz inspiratoire dans un diviseur selon l'une quelconque des revendications précédentes, comprenant l'étape de réglage de la division du volume de gaz inspiratoire demandée, l'étape de mesure et l'étape de réinitialisation ultérieure dans la boucle de rétroaction, **caractérisée en ce que** soumis à la mesure est le volume de gaz dans chacune des lignes d'inspiration, et le signal de mesure dans le contrôleur automatique est converti en signal de commande de valve de diviseur.

**14.** Le procédé de division du volume de gaz inspiratoire selon la revendication 13, **caractérisé en ce qu'**il comprend en outre l'étape de mesure du volume de gaz dans les lignes d'expiration et d'affichage de l'indication sur le circuit de surveillance.

**15.** Le procédé de division du volume de gaz inspiratoire selon la revendication 13 ou 14, **caractérisé en ce qu'**il comprend en outre l'étape de mesure de la pression de gaz dans les lignes d'expiration et d'affichage de l'indication sur le circuit de surveillance (24).

FIG.1

FIG.2

FIG.3

FIG.4

EP 3 154 617 B1

11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- PL 179784

- WO 179784 A

**Non-patent literature cited in the description**

- **DAROWSKI M. et al.** A New Control Solution for Independent Synchronous Ventilation of Lungs. *Biocybernetics and Biomedical Engineering,* 2010, vol. 30 (2